# EUROPEAN PATENT SPECIFICATION

(11) **EP 3 838 198 B1**
(45) Date of publication and mention of the grant of the patent: **17.01.2024**
(21) Application number: 20208609.6
(22) Date of filing: 19.11.2020
(51) Int. Cl.: A61B 17/70

(54) **ANTI-SPLAY HEAD AND SET SCREW FOR SPINAL FIXATION**
ANTI-SPREIZKOPF UND STELLSCHRAUBE ZUR WIRBELSÄULENFIXATION
TÊTE ANTI-ÉVASEMENT ET ENSEMBLE VIS DE FIXATION SPINALE

(30) Priority: 20.12.2019 US 201916723270
(43) Date of publication of application: 23.06.2021
(73) Proprietor: Warsaw Orthopedic, Inc., Warsaw, IN 46582 (US)
(72) Inventor: ITALIAIE, Chris, Memphis, Tennessee 38103 (US)
(74) Representative: Viering, Jentschura & Partner mbB Patent- und Rechtsanwälte

(56) References cited:
- US-A1- 2004 260 284
- US-A1- 2007 270 813
- US-A1- 2008 234 757

## Description

### FIELD

The present technology is related generally to spinal fixation systems and processes and, more particularly, to an anti-splay head and set-screw arrangement.

### BACKGROUND

Surgical screws and rods are used commonly in surgically correcting spinal abnormalities. Pedicle screw assemblies are used to facilitate placement and attachment of a spinal rod relative to the spine. Such pedicle screw assemblies include at least a bone screw section or anchor and at least one receiver portion attached to the bone screw. The bone screw sections are anchored to the vertebrae in the surgical procedure.

The receiver portions have arms between which portions of the spinal rod is received. The arms are often configured with internal threading corresponding to threading on a set screw. Following screw anchoring in the bone, and placement of the rod between the arms of the receiver, a set screw is threaded between the arms to secure the rod in the receiver.

The receiver portions of typical pedicle screws can be angularly or fixedly positionable with respect to the screw sections to facilitated desired attachment of the spinal rod between vertebrae.

Outward forces on the receiver arms over time following implantation try to splay the arms away from each other. One manner of limiting splay is by receiver and set-screw threads configured, such as with sloping thread faces, such that the set screw holds the arms inward, against the set screw.

Other attempted solutions for splaying include using a cap installable around at least a portion an exterior of the receiver arms, to hold the arms from moving away from each other.

These and other prior efforts to avoid splaying have various shortcomings. Shortcomings include undesirable cost, form factor (e.g., implant size or fit within the patient), and strength over time.

US 2004/260284 A1 describes an anti-splay pedicle screw according to the preamble of claim 1 and a method for forming an anti-splaying system according to the preamble of claim 9.

### SUMMARY

The systems, process, and techniques of this disclosure relate generally to an anti-splay head and set-screw arrangement. The invention is set out in the appended set of claims.

The present disclosure provides a system including an anti-splaying system including a receiver having a base, first and second arms, and first and second locking channels. The arms extend from the base forming a rod-receiving cavity, and arms have first and second inner threads formed in respective inner surfaces thereof. A first locking channel is formed in the inner surface of the first arm and a second locking channel being formed in the inner surface of the second arm.

The system also includes a set screw having a driving portion, such as approximal driving portion. The set screw also includes a body portion and an external thread extending form the body portion. The body is connected to the driving portion. The set screw also includes a distal locking component connected rotatably to the body portion.

The distal locking component of the set screw and the locking channel of the receiver are configured such that the locking component can, in operation of the system, slide into securing position within the locking channels. When in the secured position, the distal locking component fixes the two arms of the receiver from splaying away from each other.

In various embodiments, the locking component of the set screw has an anchor. The anchor is connected rotatably to the body portion by a slot of the body portion when the system is assembled. The anchor may be generally T-shaped, for instance.

Or, the body portion may include an anchor connected rotatably to a slot of the locking component of the set screw, in which case the anchor may be generally T-shaped.

The locking component has opposing lateral ends having sizes and shapes corresponding to size and shape of the locking channel. Each lateral end includes opposing shoulders sized and shaped to engage opposing shoulders of respective ones of the locking channels.

The first and second locking channels extend distally beyond a bottom of the first and second inner threads, respectively. The first and second locking channels extend distally beyond a bottom of the first and second inner threads, respectively, by an amount equal generally to at least a height of the locking component.

In some cases, the first locking channel extends between opposing sections of the first thread of the first receiver arm, from a first proximal opening to a first distal stop at or adjacent a distal end of the first inner thread, and the second locking channel extends between opposing sections of the second thread of the second receiver arm, from a second proximal opening to a second distal stop at or adjacent a distal end of the second inner thread. The set screw, including the locking component, has a height, and the first and second distal stops may be positioned, such that, in operation of the system, a distal surface of the locking component engages a spinal rod positioned in the cavity of the receiver, for instance.

Further, the disclosure provides a system including an anti-splaying system having a bone anchor, a spinal rod, an anti-splay receiver, and an anti-splay set screw. The receiver and set screw are in any way like the receivers and set screws described above.

The disclosure describes also a method (not claimed) for assembling an anti-splaying system, wherein the system includes any of the features described above. The method includes aligning an anti-splay set screw with an anti-splay receiver.

The aligning includes aligning opposing ends of a locking component of the set screw with opposing locking channels of the receiver. The method further includes sliding the opposing ends distally in the locking channels as outer threads of the set screw are threaded into inner threads of arms of the receiving component.

The method may include disposing the opposing ends of the locking component into the locking channels prior to engagement between the external threads and the internal threads, and moving the ends distally in the locking channels as the external threads of the set screw are threaded into the inner threads of the arms of the receiving component.

In some cases, the sliding is performed, with threading the lockingcomponent into the receiver, until a distal surface of the locking component engages a rod positioned in the rod-receiving cavity of the receiver or stops of the locking channels of the receiver.

Further, the technology includes forming of the anti-splaying system described above.

Details of the disclosure are set forth in the accompanying drawings and the description below. Other features and advantages of the techniques described in this disclosure will be apparent from the description, drawings, and claims.

### BRIEF DESCRIPTION OF DRAWINGS

FIG. 1 shows perspective views of an anti-splaying system partially disassembled, the system including an anti-splay receiver and an anti-splay set screw, according to embodiments of the present disclosure;
FIG. 2 is a cross section of the anti-splay set screw of FIG. 1 taken along line F2-F2 of FIG. 1;
FIG. 3 is a side view of the anti-splaying system of FIG. 1;
FIG. 4 is a perspective view of the anti-splay set screw being lowered for insertion to the receiver;
FIG. 5 is the perspective view of FIG. 4 with the anti-splay set screw being threaded to final position within the anti-splay receiver;
FIG. 6 is a bottom view of the anti-splay set screw;
FIG. 7 is a top view of the anti-splay receiver; and
FIG. 8 is the top view of FIG. 7, with the anti-splay set screw inserted into the anti-splay receiver.

### DETAILED DESCRIPTION

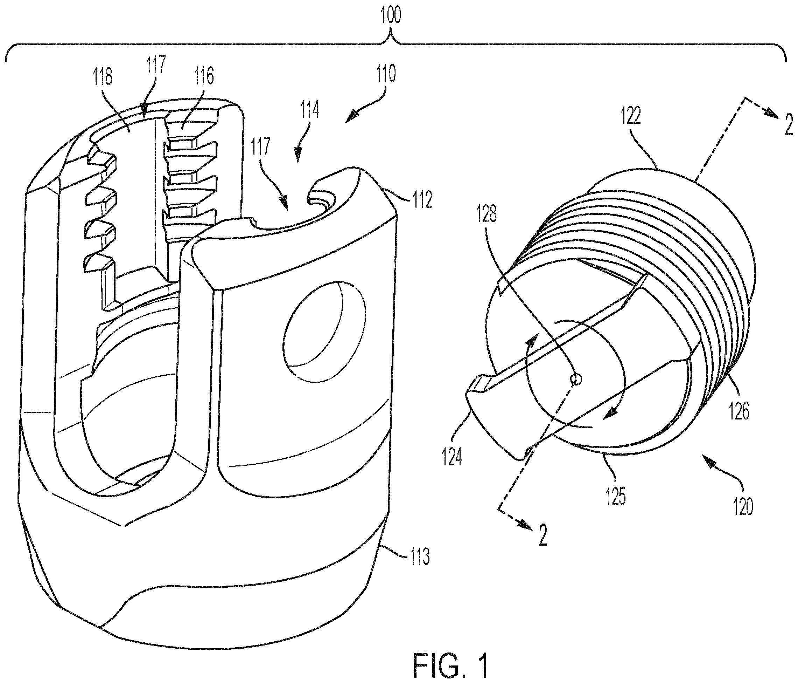

Turning now to the drawings, and more particularly to the first figure, FIG. 1 shows a perspective view of components of an anti-splay system, indicated generally by reference numeral 100.

The system 100 includes an anti-splay head, or receiver 110, and an anti-splay set screw 120, according to embodiments of the present disclosure.

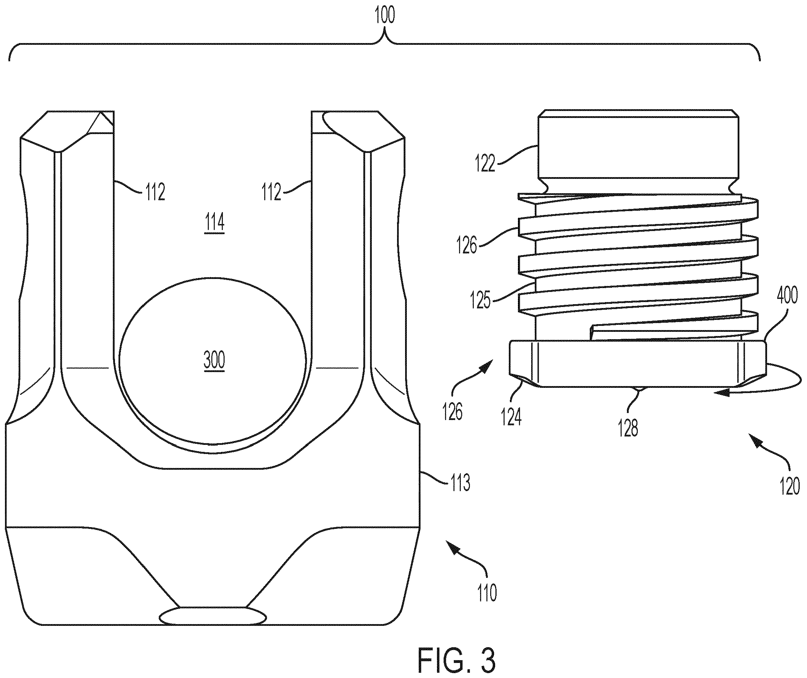

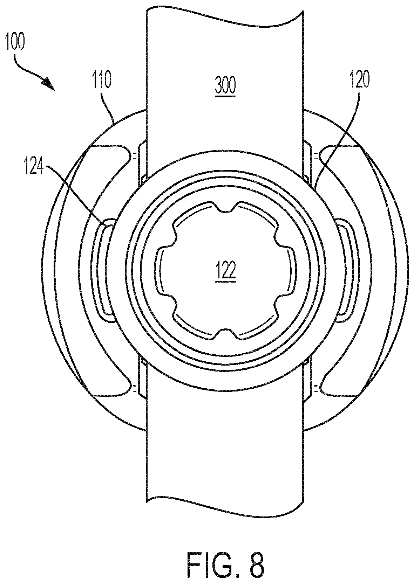

The receiver 110 has arms 112 extending up, or proximally, from a base 113. The arms 112 define a rod cavity 114 between them, for receiving a spinal-fusion rod. A rod 300 is shown schematically, disposed in the cavity 114, in FIGs. 3 and 8.

The set screw 120 extends from a driving, or proximal, end having a driving portion 122, to an anti-splay, distal, end having a rotatable locking component 124. In a contemplated embodiment, the driving portion 122 can be formed integrally or monolithically with or in the body 125 of the set screw 120. The driving portion 122 includes a driving feature 123, such as a cavity sized and shaped corresponding to any available set-screw driver (now shown).

The set-screw 120 further includes a primary body 125 extending between proximal and distal ends. The body 125 has a threaded exterior 126. The threaded surface 126 corresponds in size and shape to a thread 116 of the receiver 110, for being threaded into the receiver.

The locking component 124 is rotatably connected to the body 125, as indicated by arrows in FIG. 1. While the arrows indicate a first direction of rotation, the connecting features are in various embodiments configured so that the locking component 124 can rotate in both direction, or in one direction or the other, only.

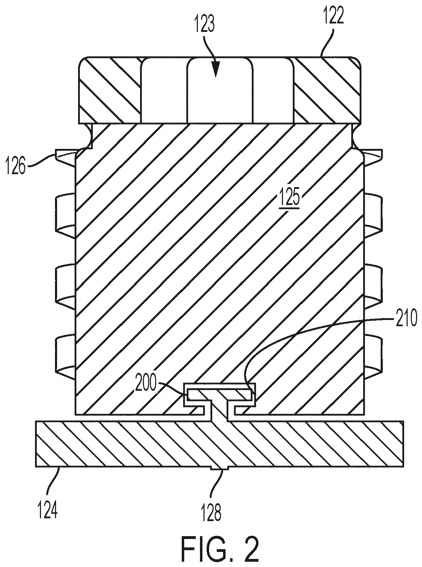

Reference numeral 128 indicates schematically in FIG. 1 a portion of a rotatable connection between the locking component 124 and the set-screw body 125. The connection is described further below in connection with FIG. 2 and other figures.

Each arm 112 has an internal thread set 116. And each thread set 116 is interrupted or separated by an anti-splay, or locking, channel 118. The threads 116 oppose each other across the cavity 114, and can be referred to as opposing threads or thread sections.

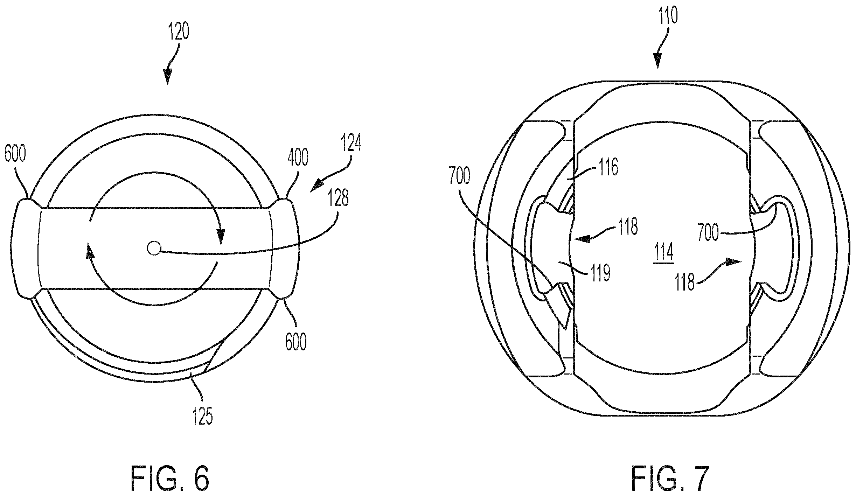

Each locking channel 118 extends distally from a proximal opening 117 (referenced in FIG. 1) to a distal stop or bottom 119 (FIG. 7). In operation of the system 100, the locking component 124 is slid distally with respect to the locking channel 118, as the external thread 126 of the set screw is threaded into the internal threads 116 of the receiver 110, until a distal surface 230 (FIG. 2) of the locking component 124 engages with the channel stop 119 (FIG. 7) or the spinal rod 300 (FIGs. 3 and 8).

The locking channel 118 in various embodiments extends down, or distally, by an amount sufficient to allow the set screw 120 to thread sufficiently into the receiver 110. If the locking channel 118 is not long enough, the locking component 124 would bottom out before the set screw 120 is able to thread sufficiently into the receiver 110. In various embodiments, each locking channel 118 extends distally beyond the internal thread 116 of the receiver arms 112. Benefits of the locking channel 118 having sufficient height include avoiding having unused thread, and saving on manufacturing cost, at least by not having to make receiver thread 116 that is not used.

The amount of extension may be generally equivalent to at least a height of the locking component 124, or at least generally a distance between a distal surface of the locking ends 400 and a distal-most portion of the set-screw thread 126, so that the external thread 126 of the set screw 120 engages generally an entirety of the internal thread 116 of the arms 112 of the receiver 110 when the lateral ends 400 of the locking component 124 reach bottom 119 of the locking channel 118, as the set screw 124 is threaded into the receiver 110.

In contemplated embodiments, the system 100, or a kit in which the system is provided, includes any of the components referenced. The system or kit can include any suitable components for performing the surgical procedures, including but not limited to implantable components and instruments for effecting the surgery. The system 100 can include, for instance, any of the anti-splay receiver 110, the anti-splay set screw 120, and the rod 300. The system 100 or kit can include the driver referenced, but not shown. And the system 100 or kit can include any number and size of these parts, including multiple anti-splay set screws and receivers 120, 110.

Turning to the second figure, FIG. 2 illustrates a cross section of the set screw 120, taken along line F2-F2 of FIG. 1. The view shows more detail of the rotational connection 128 between the set-screw locking component 124 and the set-screw body 125.

The connection 128 can be configured in any manner allowing the locking component 124 to rotate with respect to the body 125. In the embodiment of FIG. 2, the connection 128 includes a generally T-shaped anchor 200.

The body 125 has a generally T-shaped slot 210. The slot 210 is sized and shaped to hold the anchor 200. The components 200, 210 are sized to allow limited relative movement between the locking component 124 and set-screw body 125. By the limited relative movement, the locking component 124 and body 125 are fixed generally from being moved away from each other, along a longitudinal (distal/proximal) direction (i. e., from moving away from each other). The anchor 200 can rotate in the slot, and thereby the locking component 124 with respect to the set-screw body 125.

In a contemplated embodiment (not shown in detail), the slot is positioned instead in the locking component 124, having a proximal opening, for holding a T-shaped anchor extending distally from the set-screw body 125. This arrangement, including the T-shaped anchor and slot are considered shown inherently by the corresponding illustration of the T-shaped anchor and slot of FIG. 2.

The connection, while described in connection with at least one T-shape (e.g., a T-shaped anchor), can have any suitable configuration.

The connection can also include features facilitating the relative rotation, such as ball-bearings or a medical lubricant.

In operation of the system 100 (FIG. 1), the locking component 124 is aligned into the locking channel 118 of the receiver 110, by a surgeon or surgical robot. As the anti-splay set screw 120 is threaded into the receiver 110, by the surgeon or robot turning a driver (or, driving instrument; not shown) against the driving feature 123 of the driving portion 122, the locking component 124 slides down within the channel 118.

FIG. 3 is a side view of the anti-splay receiver 110 and set screw 120 of FIG. 1. Rotatability of the locking component 124 relative to the set-screw body 125 is indicated again by arrow.

FIG. 3 also shows schematically an end view of the rod 300 positioned in the cavity 114 of the receiver 110.

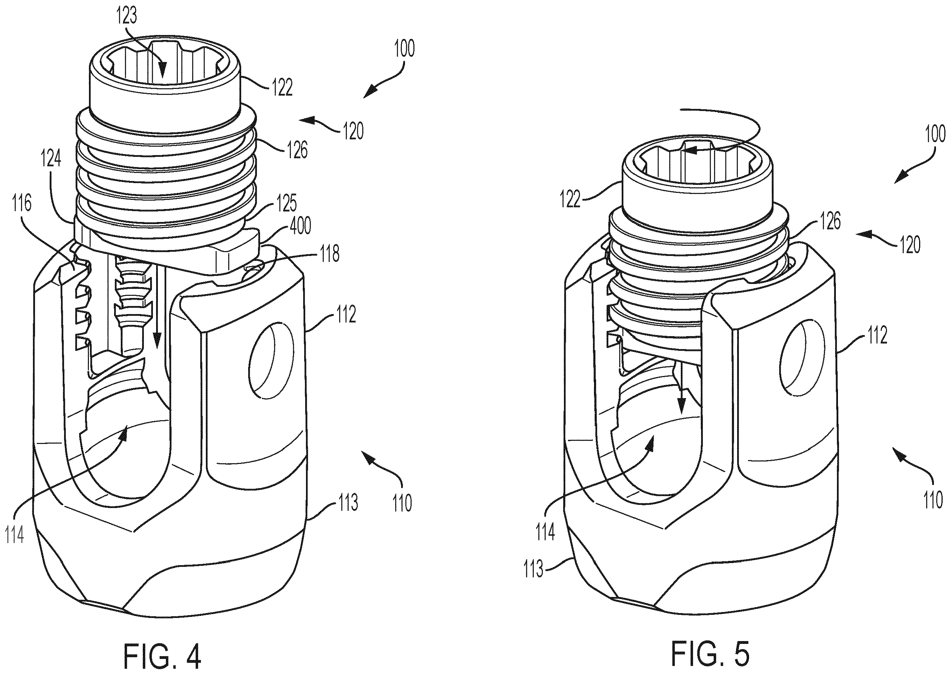

FIG. 4 is a perspective view of the anti-splay set screw 120 being lowered for insertion to the anti-splay receiver 110. The movement is indicated generally by arrow in FIG. 4.

Prior to the movement of FIG. 4, the receiver 110 is secured to a patient vertebra, by a bone screw connected to the receiver 110. The bone screw (not shown) can be any conventional or available type. The receiver 110 can be configured for toploading or bottom-loading of the bone screw into the receiver 110. The receiver 110 may in such ways be connected or connectable to the bone anchor readily-e.g., easily in the usual course of a surgery.

The anti-splay receiver 110 and anti-splay bone screw 120 can be further configured to allow desired relative motion, such as uniaxial or multiaxial motion. In some embodiments, the bone screw and the receiver 110 are fixed together, either by being formed integrally or monolithically, or by being fixedly connected to each other.

In operation of the anti-splaying system 100, as the set-screw 120 is moved toward the receiver 110, care is taken such that the locking ends 400 of the locking component 124 of the set screw 120 are aligned with the anti-splay channels 118 of the arms 112 of the receiver 110.

The anti-splay channels 118 receive ends 400 of the locking component 124. In various embodiments the locking component 124 is sized such that the ends extend further laterally than a maximum diameter of the screw body 125, and in some cases farther than a maximum diameter than the thread 126, as shown in the figures, and best seen in FIG. 2.

FIG. 5 shows the anti-splay set screw 120 having been threaded to a final position within the anti-splay receiver 110. In this position, the locking component 124 would seat against the rod 300, which is shown in FIG. 3, not FIG. 4. Motion of final threading, transitioning the set screw 120 to its final position, is indicated by arrow in FIG. 5.

As the set screw 120 is threaded into the receiver 110, the locking component 124, and particularly the ends 400 thereof, slide down in the anti-splay channels 118 of the receiver 110.

FIG. 6 is a bottom view of the anti-splay set screw 120. Relative rotatability between the set screw body 125 and the locking component 124 is indicated by arrow in FIG. 6, as in FIG. 1.

The ends 400 of the locking component 124 are configured such that, when engaged with the arms 112 of the receiver 110, being slid into the anti-splay channels 118, keep the arms 112 from moving away from each other. The connection keeps each arm 112 from moving away from the other arm-i.e., keeps the arms 118 from splaying.

The ends 400 of the locking component 124 include first engagement or locking features 600, such as shoulders. The shoulders 600, which may be referred to as external shoulders, engage second, corresponding, engagement or locking features 700 (FIG. 7) of the receiver 110. The shoulders 600 of the locking component 124 can be referred to as external shoulders, corresponding to internal shoulders 700 of the receiver 110.

The locking component 124 can be referred to as dogbone for its general shape. The configuration of each end 400 can be referred to as a double-dovetail, corresponding to the opposing shoulders 600.

In contemplated embodiments (not shown in detail), the locking or engaging features are configured in any of a variety of other ways. The features may include the locking component 124 having at each end 400 opposing internal shoulders. In this case, the internal shoulders of the locking component 124 form a lockingcomponent void, for engaging with opposing external shoulders of a protrusion extending from or on an inner surface of each arm 112 of the receiver 110, for instance. In this case, the locking component 124 is aligned so that the protrusion of the receiver 112 is received in the void of the locking component 124, as the internal shoulder of the locking component 124 is slid down along the external shoulder of the protrusion of the receiver arms 112.

FIG. 7 illustrates a plan view of the anti-splay receiver 110. The view shows a top-down, or proximately looking, view of the receiver including anti-splay channels 118.

FIG. 8 is the top view of FIG. 7, with the anti-splay set screw 120 inserted into the receiver 110, yielding the anti-splaying system 100 assembled. Full assembly in some embodiments includes the anti-splay set screw and receiver 120, 110 being connected to the bone anchor (not shown).

The rod 300 is shown seated in the anti-splay receiver 110. The rod 300 is secured in place there by the receiver 110 and the anti-splay set screw 120 threaded to the receiver 110, with the ends 400 of the locking component 124 secured in the anti-splay channel 118 of the receiver 110.

It should be understood that various examples disclosed herein may be combined in combinations other than the combinations presented specifically in the description and the accompanying drawings. It should also be understood that, depending on the example, certain acts or events of any of the processes or methods described herein may be performed in other sequence, added, merged, or left out altogether (e.g., all described acts or events may not be necessary to carry out the techniques).

Any disclosure or claim herein referencing direction is not meant to limit interpretation of the disclosure, unless the disclosure or claim requires expressly such limitation. Reference, for instance, to movement up or down herein is not limited to movement in any certain direction during surgery or system assembly, as the surgery or assembly can be performed with any of a wide variety or orientations or in any suitable reference frame.

In addition, while certain examples of this disclosure are described as being performed by a single module or unit for purposes of clarity, it should be understood that the techniques of this disclosure may be performed by a combination of units or modules associated with, for example, a medical device.

Unless defined specifically otherwise herein, all terms are to be given their broadest possible interpretation including meanings implied from the specification as well as meanings understood by those skilled in the art and/or as defined in dictionaries, treatises, etc. It must also be noted that, as used in the specification and the appended claims, the singular forms "a," "an," and "the" include plural referents unless otherwise specified, and that the terms "comprises" and/ or "comprising," when used in this specification, specify the presence of stated features, elements, and/or components, but do not preclude the presence or addition of one or more other features, steps, operations, elements, components, and/or groups thereof.

## Claims

1. An anti-splaying system (100) comprising:
a receiver (110) having a base (113), a first arm (112), a second arm (112), a first locking channel (118), and a second locking channel (118), the first arm (112) and the second arm (112) each extending from the base (113) defining a rod-receiving cavity (114) between the arms (112), the first and second arms (112) including first and second inner threads (116) formed in respective inner surfaces of the first and second arms (112), and the first locking channel (118) being formed in the inner surface of the first arm (112) and extending along a majority of the first inner threads (116), and the second locking channel (118) being formed in the inner surface of the second arm (112) and extending along a majority of the second inner threads (116); and
a set screw (120) having a driving portion (122), a body portion (125), external threads (126) extending from the body portion (125), and a distal locking component (124), the driving portion (122) being connected to the body portion (125), and the distal locking component (124) being connected rotatably to the body portion (125);
wherein the distal locking component (124) of the set screw (120) and the locking channels (118) of the receiver (110) are configured such that the locking component (124) can, in operation of the system (100), be moved into a securing position in the locking channels (118) and, when in the secured position, fixes the two arms (112) from splaying away from each other,
wherein the distal locking component (124) comprises opposing lateral ends (400) sized and shaped corresponding to size and shape of the locking channels (118), for mating engagement between the locking component (124) and the locking channels (118),
wherein the lateral ends (400) comprises opposing shoulders (600) sized and shaped to engage opposing shoulders (700) of respective ones of the locking channels (118),
wherein the first and second locking channels (118) extend distally beyond a bottom (119) of the first and second inner threads (116), respectively, by an amount equal generally to at least a height of the locking component (124), when the system (100) is assembled;
**characterized in that** the distal locking component (124) is plate shaped.

2. The system of claim 1, wherein the body portion (125) includes a distal slot (210) and the locking component (124) of the set screw (120) comprises an anchor (200) positioned at least partially in the slot (210), rotatably connecting the locking component (124) to the body portion (125).

3. The system of claim 2, wherein the anchor (200) is generally T-shaped.

4. The system of claim 1, wherein the locking component (124) includes a proximal slot and the body portion (125) comprises a distal anchor positioned at least partially in the slot, rotatably connecting the locking component (124) to the body portion (125).

5. The system of claim 4, wherein the anchor is generally T-shaped.

6. The system of one of claims 1-5, wherein the first locking channel (118) extends between opposing sections of the first thread (116) of the first receiver arm (112), and from a first proximal opening (117) to a first distal bottom (119) of the first receiver arm (112) at or adjacent a distal end of the first inner thread (116), and the second locking channel (118) extends between opposing sections of the second thread (116) of the second receiver arm (112), and from a second proximal opening (117) to a second distal bottom (119) of the second receiver arm (112) at or adjacent a distal end of the second inner thread (116).

7. The system of claim 6, wherein the set screw (120), including the locking component (124), has a height, and the first and second distal bottoms (119) are positioned, such that, in operation of the system (100), a distal surface (230) of the locking component (124) engages a spinal rod (300) positioned in the cavity (114) of the receiver (110).

8. The anti-splaying system (100) of one of claims 1-7, further comprising:
a bone anchor; and
a spinal rod (300), wherein
the receiver (110) is configured for connection with the bone anchor, the base (113) being connected or readily connectable to the bone anchor, and wherein
the set screw (120) is for securing the rod (300) in place in the receiver (110) in portion of the system (100),
wherein the distal locking component (124) of the set screw (120) and the locking channels (118) of the receiver (110) are configured such that the locking component (124) can, in operation of the system (100), slide into a securing position in the locking channels (118) and, when in the secured position, fixes the two arms (112) from splaying away from each other.

9. A method for forming an anti-splaying system (100) comprising:
forming a receiver (110) having a base (113), a first arm (112) and a second arm (112), and a first locking channel (118) and a second locking channel (118), the base (113) being connected or readily connectable to a bone anchor, the first arm (112) and the second arm (112) each extending from the base (113) forming a cavity (114) between the arms (112) for receiving the rod (300), the first and second arms (112) including first and second inner threads (116) formed in respective inner surfaces of the first and second arms (112), and the first locking channel (118) being formed in the inner surface of the first arm (112) and the second locking channel (118) being formed in the inner surface of the second arm (112), and
forming a set screw (120) having a driving portion (122), a body portion (125), external threads (126) extending from the body portion (125), and a distal locking component (124), the driving portion (122) being connected to the body portion (125), the driving portion (122) having an external thread (126), and the distal locking component (124) being connected rotatably to the body portion (125);
wherein the distal locking component (124) of the set screw (120) and the locking channels (118) of the receiver (110) are configured such that the locking component (124) can, in operation of the system (100), be moved into a securing position in the locking channels (118) and, when in the secured position, fixes the two arms (112) from splaying away from each other,
wherein the distal locking component (124) comprises opposing lateral ends (400) sized and shaped corresponding to size and shape of the locking channels (118), for mating engagement between the locking component (124) and the locking channels (118),
wherein the lateral ends (400) comprises opposing shoulders (600) sized and shaped to engage opposing shoulders (700) of respective ones of the locking channels (118),
wherein the first and second locking channels (118) extend distally beyond a bottom (119) of the first and second inner threads (116), respectively, by an amount equal generally to at least a height of the locking component (124), when the system (100) is assembled;
**characterized in that** the distal locking component (124) is plate shaped.

## Patentansprüche

1. Spreizverminderungssystem (100), umfassend:
eine Aufnahme (110), die eine Basis (113), einen ersten Arm (112), einen zweiten Arm (112), einen ersten Verriegelungskanal (118) und einen zweiten Verriegelungskanal (118) aufweist, wobei sich der erste Arm (112) und der zweite Arm (112) jeweils von der Basis (113) erstrecken, die einen stabaufnehmenden Hohlraum (114) zwischen den Armen (112) definiert, wobei der erste und der zweite Arm (112) ein erstes und ein zweites Innengewinde (116) einschließen, die in jeweiligen Innenoberflächen des ersten und des zweiten Arms (112) ausgebildet sind, und wobei der erste Verriegelungskanal (118) in der Innenoberfläche des ersten Arms (112) ausgebildet ist und sich entlang eines Großteils der ersten Innengewinde (116) erstreckt, und wobei der zweite Verriegelungskanal (118) in der Innenoberfläche des zweiten Arms (112) ausgebildet ist und sich entlang eines Großteils der zweiten Innengewinde (116) erstreckt; und
eine Schaftschraube (120), die einen Antriebsabschnitt (122), einen Körperabschnitt (125), Außengewinde (126), die sich von dem Körperabschnitt (125) erstrecken, und eine distale Verriegelungskomponente (124) aufweist, wobei der Antriebsabschnitt (122) mit dem Körperabschnitt (125) verbunden ist und die distale Verriegelungskomponente (124) mit dem Körperabschnitt (125) drehbar verbunden ist;
wobei die distale Verriegelungskomponente (124) der Schaftschraube (120) und die Verriegelungskanäle (118) der Aufnahme (110) derart konfiguriert sind, dass die Verriegelungskomponente (124), in einem Betrieb des Systems (100), in eine Befestigungsposition in den Verriegelungskanälen (118) bewegt werden kann und, wenn sie sich in der befestigten Position befindet, die zwei Arme (112) von einem Spreizen voneinander weg fixiert,
wobei die distale Verriegelungskomponente (124) gegenüberliegende seitliche Enden (400) umfasst, die entsprechend einer Größe und einer Form der Verriegelungskanäle (118) für einen Gegeneingriff zwischen der Verriegelungskomponente (124) und den Verriegelungskanälen (118) bemessen und geformt sind,
wobei die seitlichen Enden (400) gegenüberliegende Schultern (600) umfassen, die bemessen und geformt sind, um gegenüberliegende Schultern (700) von jeweiligen der Verriegelungskanäle (118) in Eingriff zu nehmen,
wobei sich der erste und der zweite Verriegelungskanal (118) distal über einen Boden (119) des ersten beziehungsweise des zweiten Innengewindes (116) um einen Betrag hinaus erstrecken, der im Allgemeinen mindestens einer Höhe der Verriegelungskomponente (124) entspricht, wenn das System (100) montiert ist;
**dadurch gekennzeichnet, dass** die distale Verriegelungskomponente (124) plattenförmig ist.

2. System nach Anspruch 1, wobei der Körperabschnitt (125) einen distalen Schlitz (210) einschließt und die Verriegelungskomponente (124) der Schaftschraube (120) einen Anker (200) umfasst, der mindestens teilweise in dem Schlitz (210) positioniert ist, der die Verriegelungskomponente (124) mit dem Körperabschnitt (125) drehbar verbindet.

3. System nach Anspruch 2, wobei der Anker (200) im Allgemeinen T-förmig ist.

4. System nach Anspruch 1, wobei die Verriegelungskomponente (124) einen proximalen Schlitz einschließt und der Körperabschnitt (125) einen distalen Anker umfasst, der mindestens teilweise in dem Schlitz positioniert ist, der die Verriegelungskomponente (124) mit dem Körperabschnitt (125) drehbar verbindet.

5. System nach Anspruch 4, wobei der Anker im Allgemeinen T-förmig ist.

6. System nach einem der Ansprüche 1 bis 5, wobei sich der erste Verriegelungskanal (118) zwischen gegenüberliegenden Bereichen des ersten Gewindes (116) des ersten Aufnahmearms (112) und von einer ersten proximalen Öffnung (117) zu einem ersten distalen Boden (119) des ersten Aufnahmearms (112) an oder angrenzend an ein distales Ende des ersten Innengewindes (116) erstreckt, und sich der zweite Verriegelungskanal (118) zwischen gegenüberliegenden Bereichen des zweiten Gewindes (116) des zweiten Aufnahmearms (112) und von einer zweiten proximalen Öffnung (117) zu einem zweiten distalen Boden (119) des zweiten Aufnahmearms (112) an oder angrenzend an ein distales Ende des zweiten Innengewindes (116) erstreckt.

7. System nach Anspruch 6, wobei die Schaftschraube (120), einschließlich der Verriegelungskomponente (124), eine Höhe aufweist und der erste und der zweite distale Boden (119) derart positioniert sind, dass in dem Betrieb des Systems (100) eine distale Oberfläche (230) der Verriegelungskomponente (124) einen Wirbelsäulenstab (300) in Eingriff nimmt, der in dem Hohlraum (114) der Aufnahme (110) positioniert ist.

8. Spreizverminderungssystem (100) nach einem der Ansprüche 1 bis 7, ferner umfassend: einen Knochenanker; und
einen Wirbelsäulenstab (300), wobei
die Aufnahme (110) für eine Verbindung mit dem Knochenanker konfiguriert ist, wobei die Basis (113) mit dem Knochenanker verbunden oder leicht verbindbar ist, und wobei
die Schaftschraube (120) zum ortsfesten Befestigen des Stabs (300) in der Aufnahme (110) in dem Abschnitt des Systems (100) angeordnet ist,
wobei die distale Verriegelungskomponente (124) der Schaftschraube (120) und die Verriegelungskanäle (118) der Aufnahme (110) derart konfiguriert sind, dass die Verriegelungskomponente (124), in dem Betrieb des Systems (100), in eine Befestigungsposition in den Verriegelungskanälen (118) gleiten kann und, wenn sie sich in der befestigten Position befindet, die zwei Arme (112) vor dem Spreizen voneinander weg fixiert.

9. Verfahren zum Ausbilden eines Spreizverminderungssystems (100), umfassend:
Ausbilden einer Aufnahme (110), die eine Basis (113), einen ersten Arm (112) und einen zweiten Arm (112) und einen ersten Verriegelungskanal (118) und einen zweiten Verriegelungskanal (118) aufweist, wobei die Basis (113) mit einem Knochenanker verbunden oder leicht verbindbar ist, wobei sich der erste Arm (112) und der zweite Arm (112) jeweils von der Basis (113) erstrecken, die einen Hohlraum (114) zwischen den Armen (112) zum Aufnehmen des Stabs (300) ausbildet, wobei der erste und der zweite Arm (112) ein erstes und ein zweites Innengewinde (116) einschließen, die in jeweiligen Innenoberflächen des ersten und des zweiten Arms (112) ausgebildet sind, und wobei der erste Verriegelungskanal (118) in der Innenoberfläche des ersten Arms (112) ausgebildet ist und der zweite Verriegelungskanal (118) in der Innenoberfläche des zweiten Arms (112) ausgebildet ist, und
Ausbilden einer Schaftschraube (120), die einen Antriebsabschnitt (122), einem Körperabschnitt (125), Außengewinde (126), die sich von dem Körperabschnitt (125) erstrecken, und eine distale Verriegelungskomponente (124) aufweist, wobei der Antriebsabschnitt (122) mit dem Körperabschnitt (125) verbunden ist, wobei der Antriebsabschnitt (122) ein Außengewinde (126) aufweist und die distale Verriegelungskomponente (124) mit dem Körperabschnitt (125) drehbar verbunden ist;
wobei die distale Verriegelungskomponente (124) der Schaftschraube (120) und die Verriegelungskanäle (118) der Aufnahme (110) derart konfiguriert sind, dass die Verriegelungskomponente (124), in dem Betrieb des Systems (100), in eine Befestigungsposition in den Verriegelungskanälen (118) bewegt werden kann und, wenn sie sich in der befestigten Position befindet, die zwei Arme (112) von einem Spreizen voneinander weg fixiert,
wobei die distale Verriegelungskomponente (124) gegenüberliegende seitliche Enden (400) umfasst, die entsprechend einer Größe und einer Form der Verriegelungskanäle (118) für einen Gegeneingriff zwischen der Verriegelungskomponente (124) und den Verriegelungskanälen (118) bemessen und geformt sind,
wobei die seitlichen Enden (400) gegenüberliegende Schultern (600) umfassen, die bemessen und geformt sind, um gegenüberliegende Schultern (700) von jeweiligen der Verriegelungskanäle (118) in Eingriff zu nehmen,
wobei sich der erste und der zweite Verriegelungskanal (118) distal über einen Boden (119) des ersten beziehungsweise des zweiten Innengewindes (116) hinaus erstrecken, um einen Betrag, der im Allgemeinen mindestens einer Höhe der Verriegelungskomponente (124) entspricht, wenn das System (100) montiert ist;
**dadurch gekennzeichnet, dass** die distale Verriegelungskomponente (124) plattenförmig ist.

## Revendications

1. Système anti-écartement (100) comprenant :
un récepteur (110) ayant une base (113), un premier bras (112), un second bras (112), un premier canal de verrouillage (118) et un second canal de verrouillage (118), le premier bras (112) et le second bras (112) s'étendant chacun à partir de la base (113) définissant une cavité de réception de tige (114) entre les bras (112), le premier et le second bras (112) comportant un premier et un second filetage interne (116) formés dans des surfaces internes respectives du premier et du second bras (112), et le premier canal de verrouillage (118) étant formé dans la surface interne du premier bras (112) et s'étendant le long d'une majorité des premiers filetages internes (116) et le second canal de verrouillage (118) étant formé dans la surface interne du second bras (112) et s'étendant le long d'une majorité des seconds filetages internes (116) ; et
une vis de réglage (120) ayant une partie d'entraînement (122), une partie de corps (125), des filetages externes (126) s'étendant à partir de la partie de corps (125), et un composant de verrouillage distal (124), la partie d'entraînement (122) étant reliée à la partie de corps (125), et le composant de verrouillage distal (124) étant relié de manière rotative à la partie de corps (125) ;
dans lequel le composant de verrouillage distal (124) de la vis de réglage (120) et les canaux de verrouillage (118) du récepteur (110) sont conçus de telle sorte que le composant de verrouillage (124) peut, lors du fonctionnement du système (100), être déplacé dans une position de fixation dans les canaux de verrouillage (118) et, lorsqu'il est en position fixée, empêche les deux bras (112) de s'écarter l'un de l'autre,
dans lequel le composant de verrouillage distal (124) comprend des extrémités latérales opposées (400) dimensionnées et formées pour correspondre à une taille et une forme des canaux de verrouillage (118), pour une mise en prise par emboîtement entre le composant de verrouillage (124) et les canaux de verrouillage (118),
dans lequel les extrémités latérales (400) comprennent des épaulements opposés (600) dimensionnés et formés pour venir en prise avec des épaulements opposés (700) de ceux respectifs des canaux de verrouillage (118),
dans lequel le premier et le second canal de verrouillage (118) s'étendent de manière distale au-delà d'un fond (119) des premier et second filetages internes (116), respectivement, à une quantité égale généralement à au moins une hauteur du composant de verrouillage (124), lorsque le système (100) est assemblé ;
**caractérisé en ce que** le composant de verrouillage distal (124) est en forme de plaque.

2. Système selon la revendication 1, dans lequel la partie de corps (125) comporte une fente distale (210) et le composant de verrouillage (124) de la vis de réglage (120) comprend un ancrage (200) positionné au moins partiellement dans la fente (210), reliant de manière rotative le composant de verrouillage (124) à la partie de corps (125).

3. Système selon la revendication 2, dans lequel l'ancrage (200) est généralement en forme de T.

4. Système selon la revendication 1, dans lequel le composant de verrouillage (124) comporte une fente proximale et la partie de corps (125) comprend un ancrage distal positionné au moins partiellement dans la fente, reliant de manière rotative le composant de verrouillage (124) à la partie de corps (125).

5. Système selon la revendication 4, dans lequel l'ancrage est généralement en forme de T.

6. Système selon l'une des revendications 1-5, dans lequel le premier canal de verrouillage (118) s'étend entre des sections opposées du premier filetage (116) du premier bras récepteur (112), et d'une première ouverture proximale (117) à un premier fond distal (119) du premier bras récepteur (112) au niveau ou adjacent à une extrémité distale du premier filetage interne (116), et le second canal de verrouillage (118) s'étend entre des sections opposées du second filetage (116) du second bras récepteur (112), et d'une seconde ouverture proximale (117) à un second fond distal (119) du second bras récepteur (112) au niveau ou adjacent à une extrémité distale du second filetage interne (116).

7. Système selon la revendication 6, dans lequel la vis de réglage (120), comportant le composant de verrouillage (124), a une hauteur, et les premier et second fonds distaux (119) sont positionnés, de telle sorte que, lors du fonctionnement du système (100), une surface distale (230) du composant de verrouillage (124) vient en prise avec une tige spinale (300) positionnée dans la cavité (114) du récepteur (110).

8. Système anti-écartement (100) selon l'une des revendications 1-7, comprenant en outre : un ancrage osseux ; et
une tige spinale (300), dans lequel
le récepteur (110) est configuré pour être connecté à l'ancrage osseux, la base (113) étant connectée ou facilement connectable à l'ancrage osseux, et dans lequel
la vis de réglage (120) sert à fixer la tige (300) en place dans le récepteur (110) dans la partie du système (100),
dans lequel le composant de verrouillage distal (124) de la vis de réglage (120) et les canaux de verrouillage (118) du récepteur (110) sont conçus de telle sorte que le composant de verrouillage (124) peut, lors du fonctionnement du système (100), coulisser dans une position de fixation dans les canaux de verrouillage (118) et, lorsqu'il est en position fixée, empêche les deux bras (112) de s'écarter l'un de l'autre.

9. Procédé de formation d'un système anti-écartement (100) comprenant :
la formation d'un récepteur (110) ayant une base (113), un premier bras (112) et un second bras (112), et un premier canal de verrouillage (118) et un second canal de verrouillage (118), la base (113) étant connectée ou facilement connectable à un ancrage osseux, le premier bras (112) et le second bras (112) s'étendant chacun à partir de la base (113) formant une cavité (114) entre les bras (112) pour recevoir la tige (300), le premier et le second bras (112) comportant un premier et un second filetage interne (116) formés dans des surfaces internes respectives du premier et du second bras (112), et le premier canal de verrouillage (118) étant formé dans la surface interne du premier bras (112) et le second canal de verrouillage (118) étant formé dans la surface interne du second bras (112), et
la formation d'une vis de réglage (120) ayant une partie d'entraînement (122), une partie de corps (125), des filetages externes (126) s'étendant à partir de la partie de corps (125), et un composant de verrouillage distal (124), la partie d'entraînement (122) étant reliée à la partie de corps (125), la partie d'entraînement (122) ayant un filetage externe (126), et le composant de verrouillage distal (124) étant relié de manière rotative à la partie de corps (125) ;
dans lequel le composant de verrouillage distal (124) de la vis de réglage (120) et les canaux de verrouillage (118) du récepteur (110) sont conçus de telle sorte que le composant de verrouillage (124) peut, lors du fonctionnement du système (100), être déplacé dans une position de fixation dans les canaux de verrouillage (118) et, lorsqu'il est en position fixée, empêche les deux bras (112) de s'écarter l'un de l'autre,
dans lequel le composant de verrouillage distal (124) comprend des extrémités latérales opposées (400) dimensionnées et formées pour correspondre à une taille et une forme des canaux de verrouillage (118), pour une mise en prise par emboîtement entre le composant de verrouillage (124) et les canaux de verrouillage (118),
dans lequel les extrémités latérales (400) comprennent des épaulements opposés (600) dimensionnés et formés pour venir en prise avec des épaulements opposés (700) de ceux respectifs des canaux de verrouillage (118),
dans lequel le premier et le second canal de verrouillage (118) s'étendent de manière distale au-delà d'un fond (119) des premier et second filetages internes (116), respectivement, à une quantité égale généralement à au moins une hauteur du composant de verrouillage (124), lorsque le système (100) est assemblé ;
**caractérisé en ce que** le composant de verrouillage distal (124) est en forme de plaque.
